# EUROPEAN PATENT SPECIFICATION

(11) **EP 1 979 101 B1**
(45) Date of publication and mention of the grant of the patent: **21.02.2018**
(21) Application number: 06846640.8
(22) Date of filing: 15.12.2006
(51) Int. Cl.: B22F 3/105, B22F 1/00

(54) **METHOD AND APPARATUS FOR LOW-TEMPERATURE PLASMA SINTERING**
VERFAHREN UND VORRICHTUNG ZUM NIEDRIGTEMPERATURPLASMASINTERN
PROCEDE ET DISPOSITIF DE FRITTAGE A BASSE TEMPERATURE AVEC UN PLASMA

(30) Priority: 15.12.2005 US 751148 P; 27.01.2006 US 763068 P; 14.12.2006 US 611010
(43) Date of publication of application: 15.10.2008
(73) Proprietor: Optomec Design Company, Albuquerque, NM 87109 (US)
(72) Inventor: RENN, Michael, J., Hudson, WI 54016 (US)
(74) Representative: Van Breda, Jacobus
(86) International application number: PCT/US2006/062154
(87) International publication number: WO 2007/070868

(56) References cited:
- WO-A1-2005/075132
- WO-A2-2006/076603
- US-A- 4 400 408
- US-A- 4 772 488
- US-A1- 2002 096 647
- US-A1- 2004 029 706
- US-A1- 2005 129 383
- US-A1- 2005 184 328

## Description

### BACKGROUND OF THE INVENTION

### Field of the Invention (Technical Field):

The present invention relates generally to the field of plasma sintering.

### Background Art:

Note that the following discussion refers to a number of publications and references. Discussion of such publications herein is given for more complete background of the scientific principles and is not to be construed as an admission that such publications are prior art for patentability determination purposes.

A plasma is a partially ionized gas consisting of ions and electrons, along with the neutral atomic and molecular species. It can be formed by applying a high frequency oscillating voltage between capacitor plates or through magnetic induction to drive an electric current in the gas. The RF field accelerates the electrons' high energy and these particles then collide with neutral atoms and molecules present in the surrounding gas. The collisions result in a variety of byproducts. Molecules dissociate into excited atomic, molecular, and ionized species, and atoms can be ionized or promoted to higher excited states. These neutral and ionized species are highly reactive and can interact chemically with surfaces and other gaseous species. Although the plasma gas as a whole is near ambient temperature, the electron gas temperature is typically around 10⁴ °K. Consequently the plasma gas can react chemically and physically with surfaces and other molecular species, yielding high temperature effects, but the thermal energy imparted to the surface is relatively low. Reactive species that are recombining or relaxing in the plasma often emit a characteristic visible glow. The plasma electron density is typically around 10¹¹ cm⁻³.

Plasma-based materials processing and surface treatments are well-known, especially in the semiconductor and electronics industry. In electronics, plasmas are commonly used for cleaning impurities or oxides from surfaces, or to physically etch a surface. In IC packaging, plasma cleaning is used to clean and activate surfaces prior to bonding, encapsulation, and die attachment. Plasmas can also be used to assist in the deposition of film forming materials through polymerization of a process gas. Plasmas are also used to cross-link polymer surfaces to provide added wear and chemical resistance to the material. Various gases are used in plasma treatments depending on the particular application. Plasma treatment gases can consist of reductive, inert, and oxidative gasses. Examples include Air, Ar, N₂, H₂, O₂, CO, NH₃, and many others. Plasma processing is typically performed in a vacuum chamber at 0.01-1 Torr, but equipment has been developed that enables plasma processing at ambient and elevated pressure and temperature conditions.

Ionized particles and free radicals within the plasma gas react with surfaces that come into contact with the plasma. The surface reactions can be physical, chemical, or a combination of physical and chemical reactions. A physical reaction occurs when high-energy ions and electrons strike a surface and impart mechanical energy to the surface atoms. The surface atoms are then ejected from the surface in a process known as sputtering or ablation. Surface contamination layers can be comprised of polymers with weak C-H bonds. Ablation typically causes the weak covalent bonds to break down. Repetitive chain scission continues until the molecular weight is small enough that the surface molecules evaporate. Continued ablation can cause the surface to etch either isotropically or anisotropically depending on the orientation of the surface and the electrode configuration of the plasma source.

A chemical reaction occurs when ions and/or neutral particles within the plasma react with surface molecules and polymers. One such reaction involves reactive species converting surface polymers to high vapor pressure products that can be pumped away. For example, plasma ashing is a process where oxygen atoms, formed by a plasma, impinge on a photoresist and break the photoresist into volatile products. The volatile products are pumped away, but the plasma does not substantially affect other inorganic surface materials, i.e. plasma ashing is selective. It is known in plasma ashing that mixtures of ions and neutrals can react with surfaces at higher rates than rates of either species alone ["Plasma Etching" edited by D.M. Manos and D.L. Flamm, Academic Press 1989]. Other types of chemical reactions can involve oxidation or reduction of surface atoms and molecules. For example, an oxygen plasma gas can react with metal atoms such as silver to form silver oxide. Similarly the reducing gas, such as H₂, can react with metal oxides, such as CuO, and reduce the oxide to a metal (Cu).

Although plasma processes are typically applied to treating surfaces, a recent technique has been developed that uses a plasma to facilitate the bulk sintering of powders. This technique is known as Spark Plasma Sintering (SPS), but it is also called Field Assisted Sintering Technique (FAST) or Pulsed Electric Current Sintering (PECS). The main characteristic of SPS is that a pulsed DC current directly passes through a graphite die, and into a powder bed that has been compacted by the die. The current pulse generates heating internal to the powder sample by preferentially heating the particles at the high resistance contact points. Most notably, very high heating or cooling rates (up to 600 °K/min) can be achieved with this method. Consequently, powder samples can be sintered through the combination of the internal heating and the external powder compression and external heating. This contrasts conventional thermal sintering processes, which take many minutes to hours. The pulsed DC current or field, powder compression, and external heating appear to be essential parts of the SPS process [for example, see J.M. Lourenco, et al, "Plasma sintering of unalloyed iron: a study of surface porosity," Materials Research, Vol. 7, No. 2, 269-275, 2004].

In the printed electronics field, conductive metal features are typically printed in the form of an ink. The inks can be applied using a variety of methods including, screen printing, stencil printing, ink jetting, doctor blading, spin coating, and spray coating. The inks can consist of nanoparticle or microparticle suspensions, metallo-organic precursors, or conductive particles in an organic medium. To maintain stability of the inks, the particles are typically encapsulated with an organic capping layer that inhibits particle agglomeration. Other organic binders, sintering aids, and adhesion promoters may also be added to the ink to facilitate particular properties in the printed product. However, because of the organic additives, the inks are typically not conductive as deposited, but rather require a post-deposition thermal treatment or sintering step.

During the thermal sintering step, organic solvents and organic capping compounds break down and are released from the deposits as volatiles. Since the organics are predominately insulating, the conductivity of the deposited material increases as the organic additives are removed. Thermal treatment also causes the particles to coalesce through sintering and melting processes. As the particles coalesce, the deposited ink converts from a loose powder bed to a mechanically ductile and conductive metal. In addition, a thermal treatment is typically necessary to activate the adhesion promoter in the ink in order to achieve robust adhesion of the ink to the surface. Thermal treatments are typically performed at temperatures of 150°C or higher for up to an hour.

For example, a method for fabricating an electronic part using a nanopaste composed of inorganic nanoparticles in a suitable carrier is disclosed in U.S. Patent No. 6,921,626. The method is capable of patterned deposition of nanopastes using ink-jet deposition. The nanopaste is treated by ambient drying, thermal heating, or radiation curing to form an electrically conductive pattern. Heating the deposited pattern to between 100° C and 200° C substantially increases the conductivity. Similarly, a method for producing an electrically conductive network on the surface of a substrate is disclosed in U.S. Patent Application 20050238804. The method includes providing an emulsion comprising nano-particles dispersed in a liquid carrier. By drying the emulsion, the liquid carrier is removed, leaving the nano-particles in the form of an electrically conductive network on the substrate. However, the method further sinters the nano-particles at a temperature of about 300° C to improve conductivity. Such step limits the types of substrates that can be used with this method to glass substrates, rigid polymer substrates, flexible polymer substrates, and combinations thereof.

Other methods for sintering paste include a method for using a low-temperature sintering conductive paste for high-density circuit printing is disclosed in U.S. Patent Application No. 20040004209. The method discloses a paste of ultrafine metal particles with diameters in the range of 1 to 100 nm in combination with metal filler particles with average diameters of 0.5 to 20 microns. The particles are uniformly dispersed in a heat-curable resin and may be deposited using screen printing, dispense printing, or ink-jet printing. Also, U.S. Patent Application No. 20040185388 discloses an ink for the manufacture of printed circuit boards. The ink is comprised of a dispersion of fine particles of a metal oxide or hydroxide, in which part of the particles are reduced to a metal by energy irradiation. The ink is deposited using an ink-jet or a fluid dispense device. Conductive features may be formed by irradiating the deposit with a laser, electron, or ion beam.

WO 2005/075132 discloses a method for producing the composite nanoparticles, and more particularly to composite nanoparticles, which can be used as a base material of a bonding material, e.g., for bonding between electrodes of a semiconductor device, and a method for producing the composite nanoparticles.

A wide variety of substrate materials of interest to electronics manufacturers and others have damage thresholds near 100°C, and generally cannot tolerate the thermal temperatures and times required to produce conductive inks. Such substrates include PET, polycarbonate, PMMA, polyethylene, epoxy, and IC chips. A low-temperature sintering process would be of great advantage in printing electronics on these substrates. In addition, when scaling to production levels, it is desirable to minimize the sintering times and enhance the ink/substrate adhesion.

### SUMMARY OF THE INVENTION

The present invention is a method as described in claim 1 for sintering conductive particles, the method comprising the steps of depositing micro- or nanoparticles on a substrate, the particles comprising an electrically conductive material at least partially coated with a nonconductive material, exposing the particles to a plasma, removing at least a majority of the nonconductive material; and sintering a plurality of the conductive particles to form a deposit. The substrate temperature never exceeds 100 °C, and preferably never exceeds approximately ambient temperature. The deposit preferably comprises an ink, and the particles are metallic and comprise microparticles or nanoparticles. The exposing step preferably comprises using one or more process gases. At least one of the process gases optionally is oxidative, in which case the method optionally further comprises the step of oxidizing the deposit, thereby increasing the resistance and/or resistivity of the deposit.

The exposing step is preferably performed at a pressure between approximately 0.1 mTorr and approximately 2000 mTorr. The exposing step optionally comprises shielding the substrate from charged particles in the plasma. The deposit preferably forms a structure selected from the group consisting of an EMI shield, an interconnect, a conductive repair, an electrode, a sensor, a resistor, and a conductive film. The depositing and exposing steps are optionally performed simultaneously, in which case the deposit may optionally comprise a three dimensional structure. The depositing step is optionally performed using aerodynamic focusing of an aerosol particle stream using a sheath gas, and the sheath gas may optionally comprise the plasma.

The resistivity of the deposit is preferably less than approximately four times the bulk resistivity of the electrically conductive material, and more preferably less than approximately three times a bulk resistivity of the electrically conductive material. The method optionally further comprises the step of heating the deposit, in which case the resistivity of the deposit is preferably less than or equal to approximately two times a bulk resistivity of the electrically conductive material.

The v method of the invention may further comprise the steps of propelling an aerosol stream of particles toward a substrate, the particles comprising an electrically conductive material at least partially coated with a nonconductive material; aerodynamically focusing the stream using a sheath gas; depositing the particles on the substrate; exposing the particles to a plasma; removing at least a majority of the nonconductive material; and sintering a plurality of the conductive particles to form a deposit. The sheath gas optionally comprises the plasma. The propelling and exposing steps are optionally performed simultaneously, in which case the deposit optionally comprises a three dimensional structure. The exposing step is optionally performed after the depositing step.

Objects, advantages and novel features, and further scope of applicability of the present invention will be set forth in part in the detailed description to follow, taken in conjunction with the accompanying drawings, and in part will become apparent to those skilled in the art upon examination of the following, or may be learned by practice of the invention. The objects and advantages of the invention may be realized and attained by means of the instrumentalities and combinations particularly pointed out in the appended claims.

### BRIEF DESCRIPTION OF THE DRAWINGS

The accompanying drawings, which are incorporated into and form a part of the specification, illustrate several embodiments of the present invention and, together with the description, serve to explain the principles of the invention. The drawings are only for the purpose of illustrating a preferred embodiment of the invention and are not to be construed as limiting the invention. In the drawings:
Figure 1a is a cross-section showing metal nanoparticles that are deposited comprising an organic capping layer which insulates particles, making the entire deposit is non-conductive;
Figure 1b is a cross-section depicting plasma ions impinging on the organic capping layer and breaking it into small volatile subgroups, where the volatile organics evaporate and are removed by a vacuum pump, causing the subgroups to evaporate as gaseous particles;
Figure 1c is a cross-section of metal nanoparticles forming a conductive structure through particle-particle contact after the organics are removed;
Figure 1d is a cross-section of highly reactive nanoparticles sintering at a low temperature to form a continuous, mechanically connected metal film;
Figure 2 is a schematic of an embodiment of a Cold Sintering™ apparatus;
Figure 3 is a graph showing the decrease in the resistance of a silver nanoparticle ink that was deposited on a 100°C, PET substrate, where the resistance drops by more than an order of magnitude after a 10 minute exposure to the plasma; the graph also shows an additional reduction in resistivity when the samples were exposed to heat after the Cold Sintering™ process, however, this improvement was minor compared with the dramatic improvement seen by using the Cold Sintering™ process as a first step, so that additional heating may not be necessary for some applications;
Figure 4 is a graph showing the reduced resistance that can be achieved by following a 250°C, 30 minute oven cure with a room temperature (Cold Sintering™) process, where the resistance improves (decreases) by about 25%; and
Figure 5 is a comparative graph showing the effect of different process gasses on the sintering rate of silver ink.

### DESCRIPTION OF THE PREFERRED EMBODIMENTS

### (BEST MODES FOR CARRYING OUT THE INVENTION)

The present invention discloses a method for low-temperature sintering of printable inks, using a plasma. The inks can be deposited using any number of deposition techniques, and can be applied to processing on materials including, but not limited to, electronic, biologic, and low-temperature substrates.

The present invention describes a method for chemically and physically modifying the properties of inks on various substrates using a plasma gas, with an emphasis on the sintering of metal nanoparticle inks on heat-sensitive substrates. The specific process uses a plasma to sinter metal nanoparticle inks at room temperatures. The process achieves a resistivity of the metal ink approaching that of the corresponding bulk metal, but at processing temperatures near room temperature. The process is called Cold Sintering™. It may be performed under normal atmospheric conditions or at reduced pressures. The plasma processing gas can consist of a variety of sources depending on the ink, but typical processing gases that are proven to be effective include ambient air and combinations of N₂, O₂, H₂, and Ar. The ink samples can be held at room temperature or below or, for enhanced sintering rates, samples may be held at elevated temperatures.

This invention preferably comprises a low temperature process in which a plasma is preferably applied to an ink deposit, making the deposit electrically conductive, mechanically ductile, and adherent to a substrate surface. Specifically, the metastable atoms and other species produced in a plasma are highly reactive. These species preferably react with the organic coatings and binders in the ink and reduce them to smaller, more volatile molecules that then evaporate, leaving behind only the metal particles. The remaining metal particles are highly conductive, and therefore the plasma process produces highly conductive deposits at ambient temperature.

The plasma process of the present invention also induces the coalescence and sintering of the metal micro- or nanoparticles. The plasma preferably strips the polymer coating from the metal particle surfaces. The individual, cleaned nanoparticles typically have very high surface energy; consequently there is a high thermodynamic potential driving the particle to a lower surface energy state. A particle can achieve a lower surface energy by combining with adjacent particles. This interaction typically occurs initially by diffusion of surface atoms to form neck regions between the particles. Particles connected by a neck have lower surface area than discrete particles, and this neck forms a conductive and ductile bridge. As the particles continue to form necking regions, the total surface area is reduced and the surface energy is relaxed. This is the main thermodynamic driving mechanism responsible for the sintering of the nanoparticles, and because the surface energy of nanoparticles is typically so high, the sintering occurs even at low temperature. Nanoparticle sintering is observed experimentally by observing the reduction in resistance of a metal ink and by the conversion of a powder to a ductile metal. Good ductility implies that the particles are mechanically connected and are therefore sintered. Limited sintering between micron-sized particles has also been observed as evidenced by increased conductivity and ductility. In this case the sintering is likely limited to a small amount of sintering near the particle-particle contact points. These points have a surface energy gradient and consequently the sintering will proceed by the formation of necking between the particles, which rapidly reduces the total surface energy.

As the particles coalesce by forming necks, the total surface energy is lowered and the thermodynamic potential driving the process is reduced. To continue driving the sintering to complete densification, additional energy must typically be supplied. The additional energy can be supplied by heating in an oven or with a laser, or it could also be supplied by the plasma itself, since at higher gas pressures the plasma tends to become substantially heated. The plasma gas can transfer the heat to the substrate through collisional interactions. Additional heating could also be supplied by applying electrical current, which can cause Ohmic heating at the particle junctions to further enhance the thermal sintering. The electric current could be applied in a number of ways, but it is most conveniently applied by the inductive fields already present in the apparatus used to produce the plasma. The complete sintering or complete densification of the particles may require heating well above 100°C; however, the particle sintering caused by the plasma itself is typically sufficient to produce a highly conductive and ductile material. Larger particles may require higher amounts of heating to fully sinter the particles.

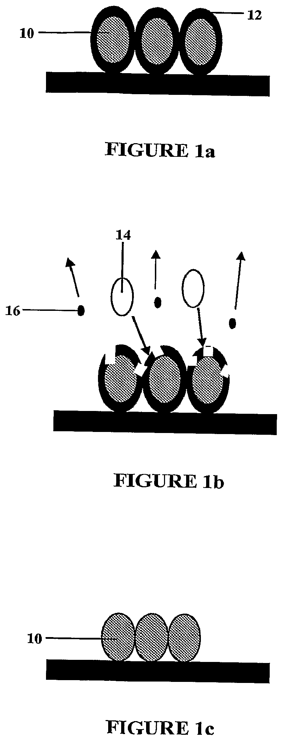

The Cold Sintering™ process is depicted in Figures 1a-d. As shown in Figure 1a, the metal nanoparticle ink is deposited to form a densely packed powder bed. Metal nanoparticles **10** comprise organic capping layer **12,** which is typically necessary for the production of stable ink suspensions. Without the organic capping agents, metal nanoparticle suspensions are unstable and prone to agglomeration. Consequently, the added organic compounds are typically an essential component of the ink. However, organic capping layer **12** is typically insulating, and consequently the ink is non-conductive immediately after deposition. Figure 1b shows a plasma gas reacting with the deposited ink and breaking the organic agents into smaller, more volatile molecules. These molecules may comprise CO, CO₂, and/or H₂O. Plasma ions **14** and neutral species preferably react preferentially with the weak carbon-hydrogen bonds in the organics. Other organic bonds are stronger and typically more difficult to break. Consequently, the long polymer chains of the organics are typically cleaved at the C-H bonds and small molecules **16** are created. The plasma reacts relatively weakly with the metal and very little etching occurs compared to the removal of the organics. Consequently, the process selectively removes organics, leaving a pure metal powder bed.

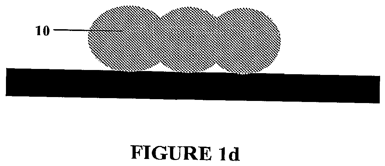

Figure 1c shows that as the organic small molecules **16** are removed, the metal nanoparticles **10** come into physical contact, and the powder bed becomes conductive. Figure 1d shows the sintering of nanoparticles **10** due to the relaxation of surface energy. This sintering occurs even at room temperature. The sintered powder has superior mechanical ductility and electrical conductivity when compared to the unsintered powder bed.

In addition to sintering the ink, the plasma process also preferably enhances the adhesion to the substrate surface. Plasma cleaning processes and surface activation are well-known in the electronics industry. Among other things, the plasma can break down weak surface groups and replace them with chemical groups from the process gas. In addition, the inherent surface cleaning and removal of oxides and organics can reduce the contact resistance between the metal ink and underlying metal surfaces. The Cold Sintering™ process of the present invention preferably drives one or more of three very important attributes in the ink: 1) sintering at low temperature never exceeding 100°C; 2) improved adhesion and wetting; and 3) reduced contact resistance to metals.

Although, the Cold Sintering™ process may take place at very low ambient plasma pressures (∼1mTorr), the addition of various process gases can greatly enhance and optimize the sintering rate. One effect of higher gas pressures is that the plasma gas becomes heated, as mentioned above. The heated plasma will transfer energy to the ink, thereby increasing the sintering rate. Higher gas pressure also increases the number of ion and neutral reactants present, which leads to enhanced reaction and sintering rates. The specific type of process gas also greatly influences the sintering rate. Inert, reductive, and oxidative gases have been used.

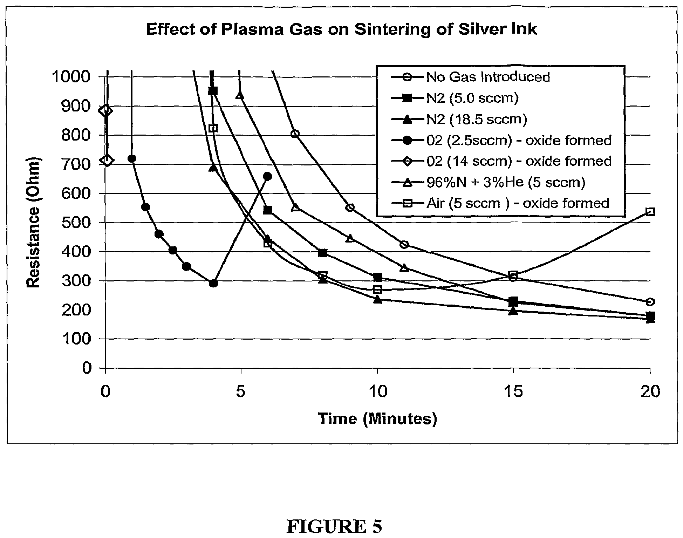

The use of inert and reductive gases typically results in approximately the same sintering rates, but an oxidative atmosphere typically results in dramatically higher rates, as shown in Figure 5. Samples can often be sintered in less than 30 seconds with an oxidative gas like O₂, compared to approximately 10 minutes for the inert gas N₂. However, if the samples are processed in O₂ for long times, silver metal becomes discolored, and the ink resistance begins to increase. It is believed that this is due to the formation of silver oxide. This oxidation of the metal is contrary to the overall goal of achieving dense metal deposits at low temperatures, but the process could be advantageous for other applications. It should be noted that the oxide formation can be reversed by slight heating of the ink samples or by applying a reductive process gas such as H₂. Overall, the most effective processing gas evaluated was a mixture of O₂ and N₂ gas, but other combinations may also be effective. The plasma treatment literature mentions a variety of gasses including Ar, N₂, O₂, CO, He, H₂, NH₃, SF₆, and CF₄.

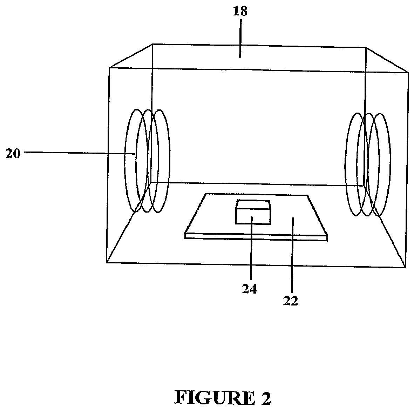

An embodiment of the Cold Sintering™ apparatus of the present invention is shown in Figure 2. The apparatus preferably comprises metal or glass chamber **18** that is evacuated to a pressure preferably between approximately 0.1 mTorr to approximately 2000 mTorr. The plasma is preferably generated by RF induction coils **20.** Other methods could also be used to generate the plasma, including but not limited to charged capacitor plates, point sources, lasers, or high-voltage electrostatic generators. Induction coils **20** may be disposed either inside or outside the chamber. The coils are preferably operated with a signal frequency of 13 MHz and a power input of 10 Watts, although any frequency and power may be used. Higher pressures generally increase the sintering rates; however, higher power is typically needed to establish a plasma at elevated pressures. Sample **24** to be sintered may be placed anywhere in chamber **18,** but preferably on sample holder **22** which is preferably situated near the center of chamber **18.** Sample holder **22** may be held at room temperature, or alternatively may be heated to increase the sintering rate. Chamber **18** preferably comprises an outlet connected to a vacuum pump and an inlet to allow process gas to be fed into the chamber.

Since the reactive species in the plasma is believed to comprise neutral species, the samples could also be placed in a region shielded from charged particles. This may be advantageous for sintering inks on substrates that are sensitive to charge buildup, such as IC chips. A simple way to shield the substrate is to enclose it in a Faraday cage, such as a wire mesh cage. The Faraday cage will block most or substantially all of the charged particles but pass the neutrals. Alternatively, the substrate could be placed in a region separated from the plasma source. The plasma could be transferred to the process zone by a flow of input gas. The charged species in plasmas are known to recombine very rapidly compared to neutral species. The system can be configured so that the substrate is downstream of the plasma so that practially all charged species are neutralized before interacting with the substate.

### Applications

The present invention is generally a method and apparatus for sintering metal inks, preferably on temperature sensitive substrates, including but not limited to plastics, thermally sensitive IC chips, and biomedical devices

### EMI Shielding and Transparent Conductive Coatings

Recent plasma display devices such as plasma TVs and computer displays have requirements that the electromagnetic radiation emitted by the device be shielded from the user. Similarly, many microwave devices, such as microwave ovens, require shielding to contain the microwave field within the device. In addition, flexible EMI shields are commonly used to protect sensitive electronic packages from ambient electromagnetic fields. These shields typically consist of a glass or plastic film with a transparent conductive coating. The shields can also consist of a fine grid or mesh of conductive wires on a support substrate. The purpose of these shields is to either absorb the electromagnetic fields emitted from the devices or to reflect the fields to protect the devices. For transparent EMI shields, the diameter of the conductive wire is typically very small (-10 microns) so that the wire has a very small optical cross section and is essentially transparent.

EMI shields can be expensive to fabricate especially if micromachining and vacuum sputtering techniques are employed. Consequently, manufacturers are exploring the possibility of printing inexpensive conductive inks onto inexpensive plastic substrates. Several printing technologies are available for printing inks such as ink-jetting, screen printing, spin coating, spray deposition, etc. Similarly, ink and surface chemistries have been developed that allow ink films to be self-assembled into fine wire and grid patterns. However, the main limitation of current production methods is in sintering the inks to achieve high quality electrical performance on plastics. The Cold Sintering™ invention provides a means to produce high quality conductive films on plastic with excellent adhesion. Since the Cold Sintering™ process occurs after the ink is printed, it is not dependent on the particular method for printing the ink.

### Interconnects

Practically all electronic devices and packages require some form of electrical interconnects to electrically connect the various electronic components. More specifically, one widespread method of electrically connecting IC chips to a circuit board is with wirebonding. However, one disadvantage of wire bonding is that the connection between the chip and board consists of a loop of wire, typically ultrasonically welded to the chip I/O and the board bond pad. This loop is mechanically fragile, and it is the source of radiative losses and signal delays at high operational frequencies.

One approach to making a better interconnect is to print a zero profile, printed ink connection between the chip I/O and the board bond pad. Several printing technologies are available for printing connections in this manner, including ink-jetting, M³D® aerosol jetting, and screen printing. The limitation to these approaches again lies in sintering the ink. When the sample is heated to sinter the ink, the differential expansion between the board and chip often causes the connection to break. In addition, the chip, board, and bonding materials typically cannot withstand the ∼200°C sintering temperature for extended periods. Since the Cold Sintering™ process occurs essentially at room temperature, the thermally induced fractures and material degradation can be eliminated.

### Repairs

Flat panel displays are currently fabricated on large glass panels with lithographic processes, which are very expensive. Any flaw, such as a broken trace, can result in the entire panel being rejected. Consequently, there is a need for a repair process to deposit and sinter metal and dielectric inks to repair flaws.

Since the panels are so large, it is not practical to heat the entire substrate to sinter the printed inks. Local heating processes are under exploration, such as laser and optical sintering, however, the required adhesion and electrical conductivity specifications have not yet been achieved. The Cold Sintering™ approach can be used to locally sinter an ink deposit at near ambient conditions. Instead of using a chamber for sintering, a small plasma source capable of operation at ambient pressure can be placed over the repair region. A repair can then be deposited and sintered in a matter of seconds.

### Biomedical Devices

Biomedical devices consist of a wide range of implantable devices, sensors, surgical and analytical tools, and catheters, for example. These devices can be constructed from a variety of plastics, moldable resins, epoxies, structural metals, and traditional electronics. The Cold Sintering™ process offers a method of embedding electronic features on or into these parts. Since the process occurs near room temperature and is largely independent of the substrate material, Cold Sintering™ can be used to sinter metal inks on a wide variety of devices. This opens the door to printing a variety of sensors, electrodes, and interconnects on devices that have a complex shape and complex material composition.

### Plastic Electronics

The emerging field of plastic electronics is seeking to print IC like electronic features on large flexible substrates. Fabrication cost is a critical driver, and currently costs are dominated by traditional thin film production methods such as sputtering and photolithography. One specific area to which Cold Sintering™ can contribute is in fabrication of high conductivity traces and electrodes on plastic film. Because of thermal damage limitations of plastic films such as PET and polycarbonate, it is difficult to sinter metal inks to give good electrical conductivity without damaging the underlying film. Rather, the plastic electronics manufacturers typically apply sputter processes to deposit metal structures, or they can deposit a conductive polymer, such as PEDOT {Poly(3,4-ethylenedioxythiophene) poly(styrenesulfonate)}. The conductive polymer is several orders more resistive than conductive metal, and consequently, it is much more desirable to deposit metal if possible.

This invention allows the sintering of pure metal inks on plastic film to achieve very high conductivity. The ink can be printed with methods such as ink jet, screen print, spin coating, doctor blading, spray coating, or the M³D® process. The Cold Sintering™ treatment can be applied to the printed inks as a batch process or it would be applied locally, to sinter only a small number of features at a time. The process is useful for producing highly conductive traces to connect discrete transistor elements to I/O. It could also be used for sintering printed gate lines or for sintering drain and source lines. The process could also be used for sintering very thin coatings of metal ink to fabricate transparent conductive films.

### Embedded Resistors

One embedded passives application is the printing of precision resistors on a circuit board. Resistor values in the range of 10 Ohms to 10 MOhms with 5% tolerance are generally of most interest. Resistors can be printed with a variety of materials, including carbon-filled epoxies, metals, and conductive oxides. The Cold Sintering™ process provides another approach to printing resistive elements. The Cold Sintering™ process has demonstrated the capability to both sinter metal particles to make highly conductive deposits and to oxidize the metal to make it non-conductive. The relative amount of oxide to metal can be adjusted by controlling the concentration of O₂ introduced, as well as controlling the process time (see, for example, Figure 5). The relative amount of oxide to metal typically determines the resistivity of the material. Consequently, it is possible to print a silver ink line and, with the Cold Sintering™ method, convert the line into a combination of metal and oxide having a precise resistance value.

### Cold Sintering™ in Real-Time

With the addition of oxygen to the system, the sintering time for silver has been demonstrated to be less than a minute. It is reasonable to expect that other enhancements are possible, such as applying external heat, laser irradiation, higher RF power, or different processing gases that will reduce the sintering time to seconds. Consequently, applying a plasma to the materials in real time, during deposition, rather than the sequential approach previously described is possible. If the plasma is being applied while material is being physically deposited, the effect would be to bond the particles together as they are being deposited, remove organic components from the ink, and sinter the materials to form a ductile deposit. Consequently, functional materials, such as conductive wires, could be deposited in a single processing step.

The simultaneous application of the plasma sintering process and particle deposition process are ideally suited for M³D® printing technology. The M³D® process deposits particles by the aerodynamic focusing of an aerosol particle stream. Consequently, it is plausible to substitute the sheath gas stream with a gas stream containing a plasma gas. The plasma gas would then flow co-axially with the aerosol particles, and as the particles are deposited on the substrate, the plasma gas would facilitate the adhesion and sintering of the particles. The plasma gas might also be directed at the deposition zone by separate gas jets that are independent of the M³D® deposition head. In such a configuration, the M³D® head could be arranged, for example, perpendicular to the substrate to deposit the particles, and the plasma gas could be directed at an angle to the deposit.

The simultaneous deposition and sintering opens the possibility of depositing three-dimensional structures. If the plasma gas is approximately coincident with the particle stream, the particles will be bonded and the organic components vaporized by the plasma sintering action. Specifically, this capability is useful for fabricating free standing metal structures such as micron-scale posts, beams, springs, bumps, etc. It would also be possible to create complex three-dimensional shapes such as dental implants. Many other applications are to be found in fabricating MEMS structures, and 3D micro-components.

### Examples

### Example 1

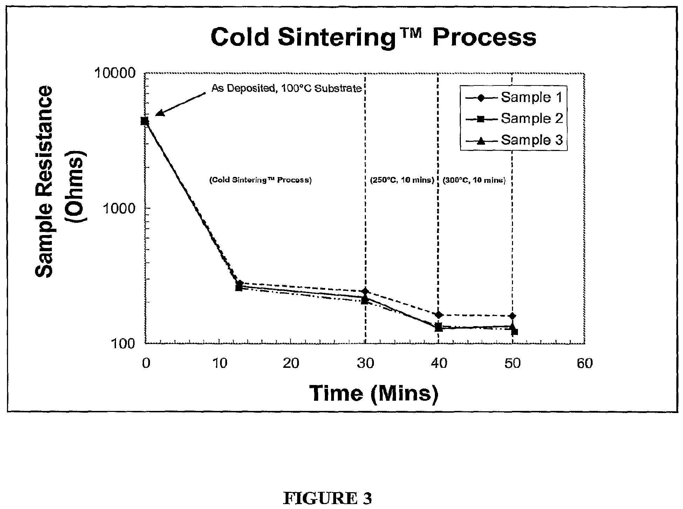

Data showing the reductions of resistance of a silver ink by the Cold Sintering™ method are shown in Figure 3. The apparatus used comprised a plasma chamber commercially available from Harrick Plasma (Harrick Plasma model PDC 32G). Samples were initially prepared by printing silver ink onto a 100°C heated glass slide. The ink deposits are slightly conductive immediately after printing on the glass and show an initial resistance of approximately 4400 Ohms. When the samples are exposed to the plasma at approximately 100mTorr chamber pressure with no external input gas, the resistance falls very rapidly to approximately 280 Ohms in about ten minutes. Additional Cold Sintering™ treatment for longer time periods continues to reduce the resistance, but with diminishing rate. After treating the samples with the Cold Sintering™ process for 30 minutes, the resistance falls slightly more, to about 244 Ohms. The samples were then heated in an oven at 250°C for 10 minutes. This thermal treatment further reduced the resistance by approximately 30% to 164 Ohms, or approximately twice the equivalent bulk resistance of silver. Additional baking above 250°C yielded only a slight decrease in the resistance value. From this study, it is concluded that the plasma sintering method can reduce the resistance of the ink to within 30% of the value that can be obtained by high temperature treatment at 300°C. However, the plasma treatment occurs at low temperatures.

### Example 2

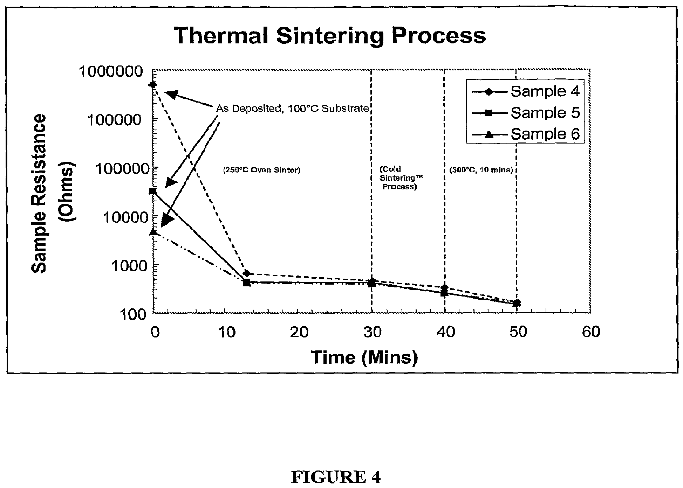

Figure 4 shows the improved resistance that can be achieved by exposing samples to a plasma after initially baking the sample at 250°C. In this study, silver ink is again printed on glass slides heated to 100°C. The three ink samples are conductive immediately after printing, with resistance values ranging from a few kOhms to several MOhms. After baking at 250°C for 30 minutes, the resistance of all samples drops to around 445 Ohms. After the oven treatment, the samples were then treated with the Cold Sintering™ process for 10 minutes, and the resistance of the samples dropped by 25%. Finally, the samples were placed back in an oven at 300°C for 10 minutes and the resistance fell by an additional 50%. The conclusion is that the Cold Sintering™ process will further reduce the resistance of inks that have been exposed to relatively high temperature oven sintering. However, the resistance value is as good as the value achieved at the highest thermal temperatures (300°C).

The results above were obtained without additional processing gas; the plasma was formed from only residual gas in the chamber.

### Example 3

Figure 5 shows the effect of adding a processing gas to the sintering of silver ink deposits on polyimide substrate. The "No Gas" curve corresponds to the case where no external gas is introduced (chamber pressure approximately 100 mTorr). As can be seen, the resistance of the ink deposit reaches an asymptotic value in roughly 15 minutes. The addition of Nitrogen gas ("N2" curves) into the chamber at rates of 5.0 sccm to 18.5 sccm is seen to increase the sintering rate by up to a factor of two. The "O2" curves show the effect of introducing oxygen processing gas into the system. It is immediately evident that the O₂ strongly enhances the sintering rate to as little as one minute at 14 sccm. At longer exposures to O₂ gas, the ink resistance begins to increase and a black film forms as the surface of the silver ink deposits. It is believed this is caused by the formation of silver oxide on the silver metal after it has been cleaned of organic impurities. Since silver oxide is non-conductive, the ink resistance increases as it is converted to silver oxide. It is noted that the silver oxide can be converted back to silver by baking the samples at approximately 200°C.

Since the oxygen gas gives the strongest effect, but also causes formation of silver oxide, attempts have been made to introduce a mixture of two gases. The "Air" curve shows the introduction of ambient air (approximately 70% N₂ and 30% O₂) into the system at 5.0 sccm. The sintering rate for air is approximately equivalent to the sintering rate achieved for a flow rate of 18.5 ccm of pure N₂, which demonstrates that the 30% oxygen content in air strongly increases the sintering rate. Similarly, the sintering rate for air at 5.0 seem is lower than the sintering rate using pure O₂ at 2.5 sccm. This is consistent with the fact that the oxygen concentration contained in ambient air at 5 sccm is lower than that of pure oxygen gas at 2.5 sccm. For extended sintering periods, the resistance increases for all the oxygen-rich processing gases. The tendency of oxygen to react with silver and silver oxide counteracts the sintering process. However, it is speculated that the reductive gasses such as CO or H₂ could be introduced to inhibit AgO formation, or the sample might be heated slightly during sintering so that the oxide becomes unstable. It is worth noting that certain noble metals such as gold do not form stable oxides and consequently they can be sintered in the oxygen-rich environment.

## Claims

1. A method for sintering metal particles, wherein the particles are microparticles or nanoparticles, the method comprising the steps of:
depositing the micro- or nanoparticles on a substrate, the particles comprising a metallic material at least partially coated with an electrically nonconductive organic capping layer;
exposing the particles to a plasma;
removing at least a majority of the nonconductive organic capping layer; and
sintering the metal particles in physical contact where the electrically nonconductive organic capping layer has been removed, thereby forming a conductive deposit,
wherein the substrate temperature never exceeds 100 °C.

2. The method of claim 1 wherein a substrate temperature never exceeds approximately ambient temperature.

3. The method of claim 1 wherein the exposing step comprises using one or more process gases.

4. The method of claim 3 wherein at least one of the process gases is oxidative.

5. The method of claim 4 further comprising the step of oxidizing the deposit, thereby increasing a deposit resistance.

6. The method of claim 1 wherein the exposing step is performed at a pressure between approximately 0.1 mTorr and approximately 2000 mTorr.

7. The method of claim 1 wherein the exposing step comprises shielding the substrate from charged particles in the plasma.

8. The method of claim 1 wherein the deposit forms a structure selected from the group consisting of an EMI shield, an interconnect, a conductive repair, an electrode, a sensor, a resistor, and a conductive film.

9. The method of claim 1 wherein the depositing and exposing steps are performed simultaneously.

10. The method of claim 1 wherein the depositing step is performed using aerodynamic focusing of an aerosol particle stream using a sheath gas.

11. The method of claim 1 further comprising the step of heating the deposit.

12. The method of claim 1 or claim 11 wherein a resistivity of the deposit is less than or equal to approximately four times a bulk resistivity of the electrically conductive material.

13. The method of claim 1 further comprising the steps of:
propelling an aerosol stream of the particles toward a the substrate, and
aerodynamically focusing the stream using a sheath gas;
optionally wherein a substrate temperature never exceeds approximately ambient temperature;
optionally wherein the propelling and exposing steps are performed simultaneously;
optionally wherein the exposing step is performed after the depositing step.

14. The method of claim 10 or claim 13 wherein the sheath gas comprises the plasma.

15. The method of claim 9 or claim 13 wherein the deposit comprises a three dimensional structure.

## Patentansprüche

1. Verfahren zum Sintern von Metallpartikeln, wobei die Partikel Mikropartikel oder Nanopartikel sind, wobei das Verfahren die folgenden Schritte umfasst:
Ablagern der Mikro- oder Nanopartikel auf einem Substrat, wobei die Partikel ein metallisches Material umfassen, das mindestens teilweise mit einer elektrisch nicht leitfähigen organischen Deckschicht beschichtet ist;
Aussetzen der Partikel einem Plasma;
Entfernen mindestens einer Mehrheit der nicht leitfähigen organischen Deckschicht, und
Sintern der Metallpartikel in physischem Kontakt, wo die elektrisch nicht leitfähige organische Deckschicht entfernt wurde, wodurch eine leitfähige Ablagerung gebildet wird,
wobei die Substrattemperatur nie 100 °C übersteigt.

2. Verfahren nach Anspruch 1, wobei eine Substrattemperatur nie etwa die Umgebungstemperatur übersteigt.

3. Verfahren nach Anspruch 1, wobei der Schritt des Aussetzens das Verwenden eines oder mehrerer Prozessgase umfasst.

4. Verfahren nach Anspruch 3, wobei mindestens eines der Prozessgase oxidativ ist.

5. Verfahren nach Anspruch 4, ferner den Schritt des Oxidierens der Ablagerung umfassend, wodurch der Widerstand der Ablagerung erhöht wird.

6. Verfahren nach Anspruch 1, wobei der Schritt des Aussetzens bei einem Druck zwischen etwa 0,1 mTorr und etwa 2000 mTorr durchgeführt wird.

7. Verfahren nach Anspruch 1, wobei der Schritt des Aussetzens das Abschirmen des Substrats von geladenen Partikeln in dem Plasma umfasst.

8. vVerfahren nach Anspruch 1, wobei die Ablagerung eine Struktur bildet, die aus der Gruppe bestehend aus einer EMI-Abschirmung, einem Schaltungsträger, einer leitfähigen Instandhaltung, einer Elektrode, einem Sensor, einem Widerstand, und einer leitfähigen Folie ausgewählt ist.

9. Verfahren nach Anspruch 1, wobei die Schritte des Ablagerns und des Exponierens gleichzeitig durchgeführt werden.

10. Verfahren nach Anspruch 1, wobei der Schritt des Ablagerns unter Verwendung von aerodynamischer Fokussierung eines Aerosol-Partikelstroms unter Verwendung eines Hüllgases durchgeführt wird.

11. Verfahren nach Anspruch 1, ferner den Schritt des Erhitzens der Ablagerung umfassend.

12. Verfahren nach Anspruch 1 oder Anspruch 11, wobei ein Widerstand der Ablagerung geringer oder gleich etwa dem vierfachen eines spezifischen Widerstands des elektrisch leitfähigen Materials ist.

13. Verfahren nach Anspruch 1, ferner die folgenden Schritte umfassend:
Treiben eines Aerosol-Stroms der Partikel zu einem dem Substrat, und
aerodynamisches Fokussieren des Stroms unter Verwendung eines Hüllgases;
wobei optional eine Substrattemperatur nie etwa die Umgebungstemperatur übersteigt;
wobei optional die Schritte des Treibens und des Aussetzens gleichzeitig ausgeführt werden;
wobei optional der Schritt des Aussetzens nach dem Schritt des Ablagerns durchgeführt wird.

14. Verfahren nach Anspruch 10 oder Anspruch 13, wobei das Hüllgas das Plasma umfasst.

15. Verfahren nach Anspruch 9 oder Anspruch 13, wobei die Ablagerung eine dreidimensionale Struktur umfasst.

## Revendications

1. Procédé de frittage de particules métalliques, dans lequel les particules sont des microparticules ou des nanoparticules, le procédé comprenant les étapes suivantes :
déposer les micro- ou nanoparticules sur un substrat, les particules comprenant un matériau métallique au moins partiellement revêtu d'une couche d'encapsulation organique électriquement non conductrice ;
exposer les particules à un plasma ;
retirer au moins une majorité de la couche d'encapsulation organique non conductrice ; et
fritter les particules métalliques en contact physique où la couche d'encapsulation organique électriquement non conductrice a été retirée, formant ainsi un dépôt conducteur,
dans lequel la température du substrat ne dépasse jamais 100 °C.

2. Procédé selon la revendication 1 dans lequel une température du substrat ne dépasse jamais environ la température ambiante.

3. Procédé selon la revendication 1 dans lequel l'étape d'exposition comprend l'utilisation d'un ou de plusieurs gaz de procédé.

4. Procédé selon la revendication 3 dans lequel au moins l'un des gaz de procédé est oxydatif.

5. Procédé selon la revendication 4 comprenant en outre l'étape d'oxydation du dépôt, augmentant ainsi une résistance au dépôt.

6. Procédé selon la revendication 1 dans lequel l'étape d'exposition est exécutée à une pression comprise entre environ 0,1 mTorr (0,013 Pa) et environ 2 000 mTorr (266,6 Pa).

7. Procédé selon la revendication 1 dans lequel l'étape d'exposition consiste à protéger le substrat des particules chargées dans le plasma.

8. Procédé selon la revendication 1 dans lequel le dépôt forme une structure choisie dans le groupe constitué d'un blindage EMI, d'une interconnexion, d'une ligne de réparation conductrice, d'une électrode, d'un capteur, d'une résistance et d'un film conducteur.

9. Procédé selon la revendication 1 dans lequel les étapes de dépôt et d'exposition sont exécutées simultanément.

10. Procédé selon la revendication 1 dans lequel l'étape de dépôt est exécutée en utilisant la focalisation aérodynamique d'un flux de particules d'aérosol en utilisant un gaz de gainage.

11. Procédé selon la revendication 1 comprenant en outre l'étape de chauffage du dépôt.

12. Procédé selon la revendication 1 ou la revendication 11 dans lequel une résistivité du dépôt est inférieure ou égale à environ quatre fois une résistivité volumique du matériau électriquement conducteur.

13. Procédé selon la revendication 1 comprenant en outre les étapes suivantes :
propulser un flux d'aérosol des particules vers un substrat, et
focaliser de manière aérodynamique le flux en utilisant un gaz de gainage ;
facultativement dans lequel une température de substrat ne dépasse jamais environ la température ambiante ;
facultativement dans lequel les étapes de propulsion et d'exposition sont exécutées simultanément ;
facultativement dans lequel l'étape d'exposition est exécutée après l'étape de dépôt.

14. Procédé selon la revendication 10 ou la revendication 13 dans lequel le gaz de gainage comprend le plasma.

15. Procédé selon la revendication 9 ou la revendication 13 dans lequel le dépôt comprend une structure tridimensionnelle.
